(19) Europäisches Patentamt
European Patent Office
Office européen des brevets

(11) Veröffentlichungsnummer: **0 001 115**
**A2**

# (12) EUROPÄISCHE PATENTANMELDUNG

(21) Anmeldenummer: **78100835.4**

(22) Anmeldetag: **06.09.78**

(51) Int. Cl.²: **C 07 D 519/02**
A 61 K 31/48

(30) Priorität: **09.09.77 YU 2161/77**

(43) Veröffentlichungstag der Anmeldung:
**21.03.79 Patentblatt 79/6**

(84) Benannte Vertragsstaaten:
**BE CH DE FR GB NL SE**

(71) Anmelder: **LEK, tovarna farmacevtskih in kemicnih izdelkov, n.sol.o.**
**Celovska 135**
**YU-61000 Ljubljana(YU)**

(72) Erfinder: **Rucman, Rudolf, Dipl.-Ing.**
**Ptujska 25**
**YU-61000 Ljubljana(YU)**

(72) Erfinder: **Djordjevic, Nebojsa, Dr.**
**Rimska 12**
**YU-61000 Ljbuljana(YU)**

(74) Vertreter: **Deufel, Paul et al,**
**Patentanwälte MÜLLER-BORé-DEUFEL SCHÖN-HERTEL**
**Siebertstrasse 4**
**D-8000 München 86(DE)**

(54) **2-Bromergosin und seine Säureadditionssalze, Verfahren zu deren Herstellung sowie deren Verwendung als Heilmittel.**

(57) Die neue Verbindung 2-Bromergosin wird duch selektive Bromierung des natürlichen Alkaloids Ergosin in 2-Stellung erhalten und dient, gegebenenfalls in Form seines Säureadditionssalzes, als enteral und parenteral applizierbares Arzneimittel zur Behandlung von arteriellen Hypertensionen und von Herz-Arhythmien.

EP 0 001 115 A2

MÜLLER-BORÉ · DEUFEL · SCHÖN · HERTEL
PATENTANWÄLTE

0001115

DR. WOLFGANG MÜLLER-BORÉ
(PATENTANWALT VON 1927 - 1975)
DR. PAUL DEUFEL, DIPL.-CHEM.
DR. ALFRED SCHÖN, DIPL.-CHEM.
WERNER HERTEL, DIPL.-PHYS.

-6. SEP. 1978
L 1186 EU

# 2-Bromergosin und seine Säureadditionssalze, Verfahren zu deren Herstellung sowie deren Verwendung als Heilmittel

Gegenstand der vorliegenden Erfindung sind das neue 2-Bromergosin und seine physiologisch verträglichen Säureadditionssalze, Verfahren zu deren Herstellung sowie deren Verwendung als Heilmittel zur Behandlung der arteriellen Hypertensie und der Herz-Arhythmien.
Das erfindungsgemäße 2-Bromergosin entspricht der Formel

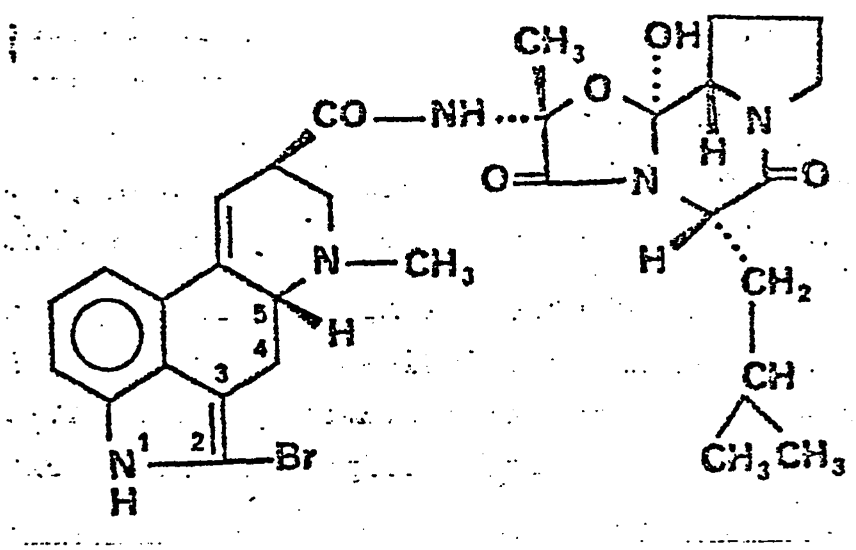

(I)

und das Verfahren zu dessen Herstellung ist dadurch gekennzeichnet, daß das natürliche Alkaloid Ergosin in 2-Stellung selektiv bromiert wird.
Die selektive Bromierung wird z.B. mit Pyrrolidon-hydrotribromid, N-Brom-Verbindungen, Dioxan-dibromid oder mit 3-Brom-4,5-dialkyl-2-oxazolidonen durchgeführt. Die Umsetzung wird bei normaler oder leicht erhöhter Temperatur in einem unte.

0001115

den Reaktionsbedingungen inerten Lösungsmittel und gegebenenfalls unter Zusatz eines Radikalinitiators [s. Bianchi G., Grünanger P., Tetrahedron 21, 817 (1965)], z.B. 2,2'-Azo-bis-(2-methylpropionitrils) in einer inerten Atmosphäre durchgeführt.

Bei der Verwendung von N-Brom-Verbindungen und Dioxan-dibromid sind die Ausbeuten mäßig. Da die Umsetzung bei Raumtemperatur mehr als 10 Stunden dauert, wird sie durch Erhitzen auf 40 bis 50°C oder durch Zusatz katalytischer Mengen eines Radikalinitiators beschleunigt. Andererseits ist ein Erhöhen der Temperatur ungünstig, da Nebenprodukte gebildet werden.

Die günstigste Bromierung bei Raumtemperatur ermöglicht Pyrrolidon-hydrotribromid. Es wird in einer Menge von 1 bis 1,5 Mol je Mol Ergosin eingesetzt. Als Lösungsmittel werden bei der Umsetzung cyclische Äther, wie Dioxan oder Tetrahydrofuran, oder chlorierte Kohlenwasserstoffe, wie Chloroform oder Methylenchlorid, verwendet.

Die Umsetzung wird praktisch so durchgeführt, daß die Ergosin-Lösung bei Raumtemperatur, in einer inerten Atmosphäre und unter Rühren mit Pyrrolidon-hydrotribromid, im gleichen Lösungsmittel gelöst, versetzt wird. Die Umsetzung ist beendet, sobald die Reaktionspartner gut vermischt werden.

Der Ausgangsstoff Ergosin ist eine bekannte natürliche Verbindung, die aus Mutterkornalkaloiden mittels Extraktion isoliert wird. Pyrrolidon-hydrotribromid ist ein Handelsprodukt, das auf dem Markt leicht zugänglich ist.

Da 2-Bromergosin nur schwach wasserlöslich ist und als solches für die therapeutische Anwendung ungeeignet ist, wird es mit einer physiologisch verträglichen organischen oder anorganischen Säure in sein Säureadditionssalz überführt. Zu diesem

Zweck können z.B. Salzsäure, Schwefelsäure, Phosphorsäure, Bromwasserstoffsäure, Ameisensäure, Essigsäure, Diäthylessigsäure, Propionsäure, Apfelsäure, Milchsäure, Fumarsäure, Maleinsäure, Methansolfonsäure, Äthansulfonsäure, Weinsäure, Zitronensäure usw. verwendet werden.

Das erfindungsgemäße 2-Bromergosin und seine physiologisch verträglichen Säureadditionssalze haben die im Folgenden beschriebenen Eigenschaften.

1. Akute Toxizität

Die mittlere lethale Dosis $DL_{50}$ wurde an Mäusen beiderlei Geschlechtes mit einem Körpergewicht von 18 bis 25 g bestimmt. Den Mäusen wurde mit einer metallischen Magensonde 2-Bromergosin-methansulfonat in einer Menge von 0,01 ml/g Körpergewicht peroral verabreicht. Die mittlere lethale Dosis, ermittelt nach der Methode nach Litchfield J.T. und Wilcoxon F., J. Pharmacol. exp. Ther. 96, 99 (1946), und das 95 %-ige Vertrauensintervall betrugen 489,7 (427,2 - 561,4) mg/kg.

2. Wirkung auf den arteriellen Blutdruck

a) Wirkung auf den Blutdruck normotensiver markotisierter Ratten

Wistar-Ratten wurden narkotisiert, die Arterie Carotis communis wurde kanüliert und der Blutdruck mittels eines Mini-Transducers auf einem Dynograph (Typ Beckmann) registriert. Die Frequenzen der Herztätigkeit wurden auf demselben Dynograph mittels eines Kardiographen registriert. 2-Bromergosin-methansulfonat wurde mittels einer Apparatur zur langsamen Infusion (B. Melsungen) in Dosen von 10, 20, 50, 150, 450, 1500 und 4500 mg/kg Körpergewicht intravenös in die Vena jugularis verabreicht.

Die Ergebnisse in der Tabelle 1 zeigen, daß durch eine

Erhöhung der Dosis eine stärkere Senkung des systolischen bzw. diastolischen Druckes erzielt wurde, während der Pulsdruck anstieg.

Tabelle 1

| Intravenöse Dosis in mcg/kg Körpergewicht | Prozentuale Veränderung des systolischen Druckes | diastolischen Druckes | Pulsdruckes |
|---|---|---|---|
| 10 | 7.41 ± 1.56 ↓ | 14.16 ± 2.68 ↓ | 13.34 ± 2.23 ↑ |
| 20 | 8.57 ± 1.54 ↓ | 17.09 ± 2.23 ↓ | 12.77 ± 6.41 ↑ |
| 50 | 11.61 ± 2.06 ↓ | 21.52 ± 2.83 ↓ | 15.43 ± 1.70 ↑ |
| 150 | 14.13 ± 1.88 ↓ | 25.33 ± 6.23 ↓ | 23.81 ± 10.86 ↑ |
| 450 | 19.46 ± 2.54 ↓ | 39.22 ± 2.48 ↓ | 33.33 ± 9.11 ↑ |
| 1500 | 22.32 ± 1.50 ↓ | 46.98 ± 1.30 ↓ | 41.49 ± 8.70 ↑ |
| 4500 | 19.38 ± 0.71 ↓ | 45.49 ± 1.93 ↓ | 37.96 ± 6.91 ↑ |

↓ = Senkung des systolischen bzw. diastolischen Druckes

↑ = Anstieg des Pulsdruckes

Alle Dosen verursachten eine Inhibition des hypertensiven Reflexes im Karotidsynus bzw. es kam zu einer Depression des Druckanstieges, der durch die einseitige Occlusion der Arterie Carotis communis hervorgerufen wurde.

b) Wirkung auf die Drucksenkung bei Spinalratten

Wistar-Ratten in Urethannarkose wurden spinalisiert (das Rückenmark wurde im Bereich des zweiten Halswirbels durchgetrennt) und auf einen Apparat für künstliche Atmung (B. Melsungen) angeschlossen. Durch diesen Eingriff wurde die zentrale Blutdruckregulierung ausgeschaltet. Bei den Tieren kam es zu einer Blutdrucksenkung von etwa 50 mm Hg. Dieser Versuch diente um festzustellen, ob die untersuchte Verbindung eine unmittelbare Wirkung auf die glatte Muskulatur der Blutgefäße besitzt.

Die Untersuchungsergebnisse haben bewiesen, daß 2-Bromergosin-methansulfonat nur bei intravenösen Dosen von 450 mcg/kg auf die Blutdrucksenkung wirkt, so daß diese in Richtung des normalen Blutdruckes erhöht wird. Die Verbindung hat eine dem Dihydroergotoxin und Dihydroergotamin ähnliche, d.h. vasokonstriktorische Wirkung, bzw. sie bewirkt die Kontraktion der glatten Muskulatur der Blutgefäße [ Rothlin E., Cerletti A., Verh. dtsch. Ges. Kreislaufforsch. 15, 158 (1949)].

c) Wirkung auf die Adrenalinhypertensie bei narkotisierten Ratten

An Wistar-Ratten, die mit Urethan narkotisiert und für die Blutdruckregistrierung zugerichtet waren, wurde im Kontrollteil des Versuches durch die intravenöse Applikation von Adrenalin in Dosen von 10 mcg/kg zunächst zweimal eine kurz dauernde Hypertensie verursacht; danach wurde 2-Bromergosinmethansulfonat in Dosen von je 10, 20, 50, 150, 450, 1500 und 4500 mcg/kg infundiert und nach 3 Min. erneut Adrenalin in gleichen Dosen wie im Kontrollteil des Versuches appliziert.

Die minimale inhibitorische Dosis für 2-Bromergosin-methansulfonat betrug nur 10 mcg/kg. Bei höheren Dosen trat die Inversion der Adrenalinwirkung ein. Die Versuche haben gezeigt, daß 2-Bromergosin-methansulfonat die Adrenalinwirkung auf den Blutdruck ungefähr im gleichen Maße wie Dihydroergotoxin bzw. Dihydroergotamin inhibiert. Diese Wirkung ist wahrscheinlich das Ergebnis des kompetitiven Antagonismus von 2-Bromergosin-methansulfonat bzw. Adrenalin gegenüber $\alpha$-adrenergen Rezeptoren [ Bricher R., Cerletti A., Helv. med. acta 16, Suppl. 22 (1949)].

d) Wirkung auf die durch Serotonin hervorgerufene Hypertensie bei Spinalratten
Es wurden spinalisierte Wistar-Ratten (bei denen das Rückenmark im Bereiche des zweiten Halswirbels durchgetrennt wurde) in Urethannarkose verwendet.

Die Versuchsergebnisse haben bewiesen, daß intravenös appliziertes 2-Bromergosin-methansolfonat in einer Dosis von 150 mcg/kg die Serotoninwirkung im Vergleich mit der Kontrollwirkung dieser Verbindung sichtlich herabsetzt. In dieser Dosis wirkt 2-Bromergosin-methansulfonat ähnlich wie Dihydroergotoxin und Dihydroergotamin.

e) Wirkung auf normotensive Ratten
Als Versuchstiere wurden 10 normotensive Wistar-Ratten beiderlei Geschlechtes mit einem Körpergewicht von 200 - 250 g verwendet. 2-Bromergosin-methansulfonat wurde in Dosen von 5 mcg/kg/Tag intraperitoneal appliziert und der systolische Blutdruck gemessen. Der Blutdruck, der zu Versuchsanfang 123 - 127 mm Hg betrug, nahm nach und nach ab, um 8 - 10 Tage nach Behandlungsbeginn den Wert von 95 - 105 mm Hg zu erreichen. Dieser Wert blieb bis Versuchsende bzw. 30 Tage unverändert.

f) Wirkung auf spontan hypertensive Ratten

0001115

In diesem Versuch wurden Gruppen von je 10 spontan hypertensiven Okamoto Aoki $F_{30}$-Ratten beiderlei Geschlechtes mit einem Gewicht von 200 - 250 g verwendet. 2-Bromergosin-methansulfonat wurde intraperitoneal in Dosen von 5 mcg/kg/Tag appliziert und der Blutdruck gemessen. Der systolische Blutdruck, der zu Anfang des Versuches 174 - 177 mm Hg betrug, wurde innerhalb 6 - 10 Tage auf 135 - 140 mm Hg herabgesetzt. Ungeachtet dessen, daß die Behandlung fortgesetzt wurde, blieb der Blutdruck bis zum Versuchsende bzw. 30 Tage bei diesem Wert.

g) Wirkung auf DOCA-hypertensive Ratten

Bei jungen Wistar-Ratten beiderlei Geschlechtes mit einem Gewicht von 40 g wurde Hypertensie dadurch hervorgerufen, daß an den Tieren unilaterale Nephrektomie durchgeführt wurde. Den Tieren wurde danach Standard-Induustriefutter und eine 1 %-ige Natriumcloridlösung ad libitum vorgelegt. 7 Tage nach erfolgter unilateraler Nephrektomie wurden 2-mal wöchentlich intramuskulär 38 mg/kg DOCA (Desoxycorticosteroidacetat) als 1 %-ige Mikrosuspension in einer 0,5 %-igen TWEEN 80-Lösung, welcher 0,5 % Carboxymethylcellulose zugesetzt wurde, appliziert. Nach 6-wöchiger DOCA-Applikation erreichte der systolische Blutdruck den maximalen Wert. Dann wurde mit der intraperitonealen Applikation con 2-Bromergosin-methansulfonat in Dosen von 5 mcg/kg begonnen.

Bei den Tieren der Kontrollgruppe blieb der Blutdruck nur 2 - 3 Wochen erhöht, um danach auf 135 mm Hg abzusinken. Bei den mit 2-Bromergosin-methansulfonat behandelten Ratten dagegen kam es zur Blutdrucksenkung bereits zu Beginn der Behandlung. Innerhalb von 10 - 12 Tagen sank der Blutdruck vom Anfangswert von 189 - 187 mm Hg im Verhältnis

BAD ORIGINAL

zur Kontrollgruppe auf seinen niedrigsten Wert von etwa 135 - 140 mm Hg. Die Differenz zwischen den beiden Gruppen wurde danach wegen der o.e. spontanen Blutdrucksenkung bei der Kontrollgruppe kleiner.

3. Wirkung von 2-Bromergosin auf das Herz

Bei Versuchen mit normotensiven narkotisierten intakten und spinalen Ratten bewirkte 2-Bromergosin-methansulfonat in wirksamen Dosen eine Bradykardie. Diese Wirkung wurde bei allen bekannten Mutterkornalkaloiden bzw. deren Derivaten festgestellt [Rothlin E., Wien, Klin. Nachr. 62, 893 (1950)].

Die Wirkung von 2-Bromergosin-methansulfonat auf das Herz in vitro wurde am isolierten Herz eines Meerschweinchens nach der modifizierten Langendorffschen Methode [Zalar S., Bano M., Djordjević N., Kozjak F., Boll. Chim. Farmac. 114, 146 (1975)] untersucht. 2-Bromergosin-methansulfonat wurde mit Hilfe einer Apparatur zur langsamen Infusion (B. Melsungen) verabreicht. Die Ergebnisse sind aus der folgenden Tabelle 2 ersichtlich.

Tabelle 2

| Verbindung | Dosis in mcg/g Herz | Maximale positive inotrope Wirkung | Maximale negative chronotrope Wirkung |
|---|---|---|---|
| 2-Bromergosin-methansulfonat | 0,70 | 80 % | 29 % |
| Dihydroergotoxin | 0,85 | 7 % | 5 % |
| Dihydroergotamin | 0,72 | 60 % | 10 % |
| Ergotamin | 0,65 | 75 % | 30 % |

Aus den dargestellten Ergebnissen ist ersichtlich, daß 2-Bromergosin auf isoliertes Herz eine Wirkung ausübt, die qualitativ und quantitativ der Ergotaminwirkung ähnlich ist.

Aufgrund der beschriebenen pharmakologischen Eigenschaften wurde festgestellt, daß

2-Bromergosin eine hypetensive Wirkung besitzt, wie durch Versuche an normotensiven Raten, an spontan hypertensiven Ratten und an DOCA-hypertensiven Ratten bewiesen wurde,

2-Bromergosin auf die Kontraktion der Kerzkammer (positive inotrope Wirkung) und auf die Herzfrequenz (negative chronotrope Wirkung) wirkt, und daß

2-Bromerergosin den durch Adrenalin und Serotonin bewirkten Blutdruck-Anstieg inhibiert.

Aufgrund dieser Eigenschaften kann 2-Bromergosin als Arzneimittel zur Behandlung von arterieller Hypertension und von Herzarythmie verwendet werden.

2-Bromergosin kann in Form seiner physiologisch verträglichen Säureadditionssalze als Arzneimittel zur enteralen und parenteralen Anwendung eingesetzt werden. Zur Herstellung von pharmazeutischen Zubereitungen werden anorganische und organische Adjuvantien zugesetzt. Für Tabletten oder Dragées werden z.B. Laktose, Stärke, Talk, Stearate usw., für Lösungen und Suspensionen z.B. Wasser, Alkohole, Glyzerin, pflanzliche Öle usw., für Suppositorien z.B. natürliche und gehärtete Öle sowie Wachse zugesetzt. Die Zubereitungen können ferner geeignete Konservierungsmittel, Stabilisatoren, oberflächenaktive Stoffe, Lösich-

BAD ORIGINAL

keitsvermittler, Süßstoffe sowie Farbstoffe enthalten.

Für 2-Bromergosin in Form von seinen physiologisch verträglichen Säureadditionssalzen beträgt eine geeignete Dosis für die intravenöse Applikation 0,005 - 0,02 mg/kg Körpergewicht und für die perorale Applikation 0,05 - 0,3 mg/kg Körpergewicht täglich, berechnet als reines 2-Bromergosin.

Die Erfindung wird durch folgende, jedoch in keiner Weise einschränkende Beispiele näher erläutert:

<u>Beispiel 1</u>

9,54 g (17,4 mMol) Ergosin werden in 180 ml Dioxan gelöst und die Lösung wird unter Rühren in einer inerten Atmosphäre mit 3,52 g (19,8 mMol) N-Bromsuccinimid, gelöst in 60 ml Dioxan, versetzt. Man läßt 10 Min. bei 40°C reagieren. Dann wird das Reaktionsgemisch in Vakuum eingedampft. Der Rückstand wird in einem Scheidetrichter zwischen 200 ml einer wässrigen 2 n Natriumcarbonatlösung und 300 ml Methylenchlorid verteilt. Die Methylenchlorid-Extrakte werden vereinigt und in Vakuum getrocknet. Der Rückstand wird in 40 ml Methylenchlorid gelöst und über eine mit neutralem Aluminiumoxyd (Aktivität II - III nach Brockmann) gefüllte Säule chromatographiert. Es wird mit Methylenchlorid, welches 0,2 % Äthanol enthält, eluiert. Die 2-Bromergosin enthaltenden Fraktionen werden in Vakuum getrocknet. Die trockene Substanz wird aus Äthylacetat kristallisiert. Es werden 4,33 g (34,8 % d. Theor.) reines, kristallinisches 2-Bromergosin mit der Zsammensetzung $C_{30}H_{36}N_5O_5Br.CH_3COOC_2H_5$ erhalten. Smp. 183° - 185°C (Zers.), spez. Drehung $[\,]_{D}^{20}$ = -91,6° (c = 1, Chloroform).

Elementaranalyse für $C_{30}H_{36}N_5O_5Br$

gefunden: C 57,63 % H 6,06 % N 10,84 % Br 12,40 %
berechnet: C 57,51 % H 5,79 % N 11,18 % Br 12,73 %

Beispiel 2

5,47 g (10 mMol) Ergosin werden in 200 ml Dioxan gelöst und die Lösung wird unter Rühren bei Raumtemperatur in einer inerten Atmosphäre mit einer Lösung von 6,94 g (14 mMol) Pyrrolidon-hydron-tribromid in 1500 ml Dioxan versetzt. Die Umsetzung ist beendet, sobald die beiden Reaktanten vermischt werden. Das Reaktionsgemisch wird in Vakuum getrocknet. Der Rückstand wird ittels Verteilung zwischen einer wässrigen und einer organischen Phase und Säulenchromatographie, wie in Beispiel 1 beschrieben, gereinigt.
Es werden 5,81 g bzw. 81,3 % d. Theor. 2-Bromergosin in Form von Äthylacetat-Kristallen erhalten. Die Verbindung hat die gleichen Eigenschaften wie in Beispiel 1.

Beispiel 3

5,47 (10 mMol) Ergosin werden in 100 ml Methylenchlorid gelöst und die Lösung wird unter Rühren -ei Raumtemperatur in einer inerten Atmosphäre mit der Lösung von 6,94 g (14 mMol) Pyrrolidon-hydrotribromid in 300 ml Methylenchlorid versetzt. Das Reaktionsgemisch wird auf die Hälfte des ursprünglichen Volumens eingeengt und 3-mal mit je 200 ml 2 n Natriumcarbonat extrahiert. Die Methylenchlorid-Lösung wird direkt über eine Kieselgelsäule chromatographiert und mit Methylenchlorid, dem nach und nach 0 bis 20 % Dioxan zugesetzt werden, eluiert. Die Fraktionen, die 2-Bromergosin enthalten, werden getrocknet und aus Äthylacetat kristallisiert. Es werden 5,06 g bzw. 70,7 % d.Theor. 2-Bromergosin in Form von Äthylacetat-Kristallen erhalten. Die Verbindung besitzt die gleichen Eigenschaften wie in Beispiel 1.

Beispiel 4

2,42 g (3,86 mMol) amorphes 2-Bromergosin (Fällung aus Petroläther) werden in 20 ml Äthanol, welches 0,22 ml (4,24 mMol) Methansulfonsäure enthält, gelöst und unter Rühren in 400 ml Diäthyläther gegossen. Nach der Filtration und Trocknung des Niederschlages werden 2,49 g bzw. 89,4 % d.Theor. an gut wasserlöslichem 2-Bromergosin-methansulfonat erhalten. Smp. 191 - 192°C, spez. Drehung $[\alpha]_D^{20}$ = +104° (c = 1, Chloroform).

Beispiel 5

Tabletten

| Zusammensetzung | mg/Tablette |
|---|---|
| 2-Bromergosin-methansulfonat | 20 |
| Laktose | 228 |
| Stärke | 27 |
| Talk | 13 |
| Traganth | 10 |
| Magnesiumstearat | 2 |
| Zusammen | 300 mg |

Der Wirkstoff wird nach Standardmethoden mit den anderen Bestandteilen vermischt, granuliert und zu Tabletten verpreßt.

Beispiel 6

Kapseln

| Zusammensetzung | mg/Kapsel |
|---|---|
| 2-Bromergosin-methansulfonat | 20 |
| Laktose | 280 |
| Zusammen | 300 mg |

Der Wirkstoff wird nach Standardmethoden mit Laktose vermischt und in Kapseln gefüllt.

0001115

Beispiel 7

Injektionslösung

| Zusammensetzung | Gewicht in mg |
|---|---|
| 2-Bromergosin-methansulfonat | 1,00 |
| Natriumcarboxymethylcellulose | 1,50 |
| Polyvinylpyrrolidon | 5,50 |
| Lecithin | 3,20 |
| Benzylalkohol | 0,01 |
| Puffer | q.s. |
| bidestilliertes Wasser | 1 ml |
| Zusammen | 1 ml |

Die Lösung wird nach Standardmethoden zubereitet, sterilisiert und in Ampullen gefüllt.

PATENTANSPRÜCHE

1. 2-Bromergosin der Formel

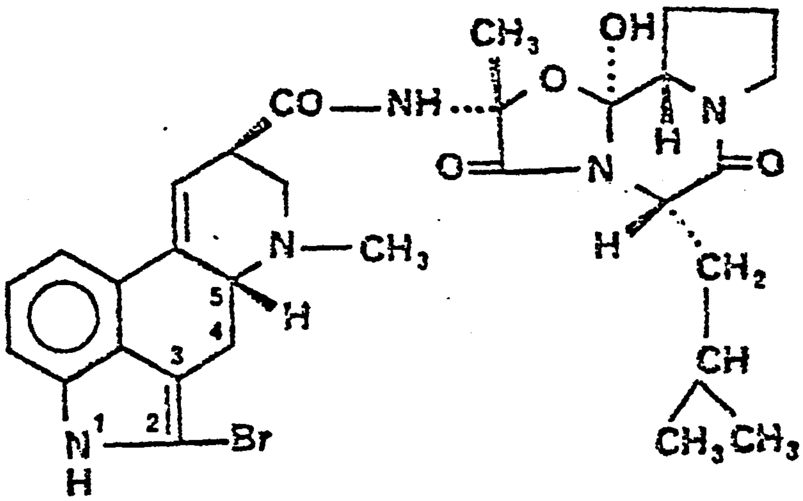

(I)

und seine physiologisch verträglichen Säureadditionssalze.

2. Verfahren zur Herstellung von 2-Bromergosin der Formel I nach Anspruch 1 und seiner physiologisch verträglichen Säureadditionssalze, dadurch gekennzeichnet, daß das natürliche Alkaloid Ergosin selektiv in der 2-Stellung bromiert wird und das erhaltene Produkt in sein physiologisch verträgliches Säureadditionssalz überführt wird.

3. Arzneimittel zur Behandlung arterieller Hypertension und von Herzarhythmien, dadurch gekennzeichnet, daß es eine pharmakologisch wirksame Menge an 2-Bromergosin der Formel I nach Anspruch 1 oder seines physiologisch verträglichen Säureadditionssalzes.

4. Verfahren zur Behandlung arterieller Hypertension und von Herzarhythmien, dadurch gekennzeichnet, daß eine pharmakologisch wirksame Menge an 2-Bromergosin der Formel I nach Anspruch 1 oder seines physiologisch verträglichen Säureadditionssalzes appliziert wird.